# EUROPEAN PATENT APPLICATION

(11) **EP 4 696 731 A1**
(43) Date of publication of application: **18.02.2026**
(21) Application number: 25745361.3
(22) Date of filing: 23.01.2025
(51) Int. Cl.: C08G 63/06, C08G 63/78, C08G 63/88, C08J 3/12, C12P 7/62, C08G 63/89, C08G 63/90, C12P 7/52, C12P 7/40

(54) **ORGANIC ACID PREPOLYMER COMPOSITION, ORGANIC ACID POLYMER, AND METHOD FOR PREPARING SAME**

(30) Priority: 24.01.2024 KR 20240010984; 04.10.2024 KR 20240134813; 07.11.2024 KR 20240157262; 29.11.2024 KR 20240175138; 06.01.2025 KR 20250001746
(71) Applicant: LG Chem, Ltd., Seoul 07336 (KR)
(72) Inventor: LEE, Jungyong, Daejeon 34122 (KR); NAM, Gibok, Daejeon 34122 (KR); JEONG, Dae Youn, Daejeon 34122 (KR); LEE, Hee Cheon, Daejeon 34122 (KR); CHOE, Dongcheol, Daejeon 34122 (KR); JEONG, Cheolhwan, Daejeon 34122 (KR); CHOE, Jae Hoon, Daejeon 34122 (KR); KIM, Chul Woong, Daejeon 34122 (KR)
(74) Representative: Plasseraud IP
(86) International application number: PCT/KR2025/001339
(87) International publication number: WO 2025/159537

(57) **Abstract**

The present invention relates to a method for preparing an organic acid prepolymer and an organic acid polymer by polymerizing and washing an organic acid composition of low purity containing an inorganic compound, and to an organic acid prepolymer composition and an organic acid polymer.

## Description

### FIELD OF THE INVENTION

### CROSS-REFERENCE TO RELATED APPLICATION(S)

This application claims the benefit of priority based on Korean Patent Application No. 10-2024-0010984 filed on January 24, 2024, Korean Patent Application No. 10-2024-0134813 filed on October 4, 2024, Korean Patent Application No. 10-2024-0157262 filed on November 7, 2024, Korean Patent Application No. 10-2024-0175138 filed on November 29, 2024, Korean Patent Application No. 10-2025-0001746 filed on January 6, 2025, and Korean Patent Application No. 10-2025-0010162 filed on January 23, 2025, and all content disclosed in the documents of the said Korean patent applications is included as part of this specification.

The present invention relates to an organic acid prepolymer composition of high purity, an organic acid polymer, and a method for preparing the same.

### BACKGROUND OF THE INVENTION

An organic acid is a commercially important chemical material having various uses in the food, cosmetic, pharmaceutical, and polymer industries. Representative organic acids include lactic acid, 3-hydroxypropionic acid (3-HP), and the like, and among them, 3-hydroxypropionic acid (3-HP) is a substance that may be utilized as a raw material for the preparation of acrylic acid, 1,3-propanediol, acrylamide, malonic acid, a biopolymer, and the like.

The production of an organic acid is largely accomplished by two methods, a chemical method and a biological method, but the chemical method has been pointed out as being non-eco-friendly due to the high cost of starting materials and the generation of toxic substances during the production process, and thus, an eco-friendly bioprocess is in the spotlight.

In the preparation of an organic acid by microbial fermentation, since other by-products are also produced together with the organic acid such as 3-hydroxypropionic acid in the process of fermenting a microorganism, a purification process such as electrodialysis and an ion exchange resin is required to extract and separate the organic acid from a fermentation broth, and there is a problem in that the process of performing this purification process is long and the process cost is also high. Accordingly, there is a need for a method to efficiently convert an organic acid of low purity containing by-products into a raw material for a biopolymer or the like.

### SUMMARY OF THE INVENTION

### TECHNICAL PROBLEM

The present invention relates to a method for preparing an oligomer, a prepolymer, and a polymer of high purity and high molecular weight from an organic acid composition of low purity containing an inorganic compound, efficiently and at a low process cost.

### TECHNICAL SOLUTION

In this specification, there is provided a method for preparing an organic acid prepolymer, comprising: a step of preparing an oligomer and/or a prepolymer by polymerizing an organic acid composition containing an inorganic compound; and a step of washing the oligomer and/or the prepolymer with a washing solution.

In addition, in this specification, there is provided a method for preparing an organic acid polymer, comprising: a step of preparing an oligomer and/or a prepolymer by polymerizing an organic acid composition containing an inorganic compound; a step of washing the oligomer and/or the prepolymer with a washing solution; and a step of preparing a polymer by polymerizing the washed oligomer and/or prepolymer.

In addition, in this specification, there is provided an organic acid prepolymer composition comprising an organic acid prepolymer in which an organic acid composition containing an inorganic compound is polymerized, wherein the inorganic compound is removed by washing.

In addition, in this specification, there is provided an organic acid polymer in which the organic acid prepolymer composition is polymerized.

Hereinafter, an organic acid prepolymer composition, an organic acid polymer, and a method for preparing the same according to specific embodiments of the invention will be described in more detail.

The terminology used in this specification is used only to describe exemplary embodiments, and is not intended to limit the present invention. A singular expression includes a plural expression unless the context clearly indicates otherwise. In this specification, terms such as "comprise," "be equipped with," or "have" are intended to designate the presence of the features, numbers, steps, components, or combinations thereof which are described, and should be understood as not precluding the possibility of the presence or addition of one or more other features, numbers, steps, components, or combinations thereof.

Since the present invention is capable of various modifications and can have various forms, specific embodiments will be illustrated and described in detail below. However, this is not intended to limit the present invention to specific disclosed forms, and it should be understood that the invention includes all modifications, equivalents, and substitutes included in the spirit and technical scope of the invention.

In addition, unless the steps constituting a preparation method described in this specification are specified as being sequential or continuous, or there is another special class, one step constituting one preparation method and another step are not interpreted as being limited to the order described in the specification. Therefore, the order of the constituent steps of the preparation method may be changed within a range that may be easily understood by a person skilled in the art, and in this case, changes obvious to a person skilled in the art accompanying it are included in the scope of the present invention.

In this specification, an organic acid means an organic compound having acidity, and may be, for example, an organic compound containing a carboxyl group or a sulfo group.

In this specification, an alkali metal may be a meaning including both an alkali metal and an alkaline earth metal.

According to an embodiment of the invention, there is provided a method for preparing an organic acid prepolymer, comprising: a step of preparing an oligomer and/or a prepolymer by polymerizing an organic acid composition containing an inorganic compound; and a step of washing the oligomer and/or the prepolymer with a washing solution.

Conventionally, in order to prepare a high value-added organic acid oligomer, prepolymer, and polymer having biodegradability, an organic acid fermentation broth was prepared by microbial fermentation and an organic acid of high purity, which is a reaction raw material, was recovered through a process of purifying it, but in order to recover the organic acid of high purity, a purification process of the fermentation broth, for example, electrodialysis, an ion exchange resin process, etc., was additionally performed, so that the process became complicated and the process operating cost was also high.

Accordingly, the present inventors have studied a process for preparing an organic acid oligomer, prepolymer, and polymer of high purity without purifying a microbial fermentation broth by a purification process such as a complicated or high process operating cost electrodialysis or ion exchange resin process, and when an oligomer and/or a prepolymer is prepared by polymerizing an organic acid composition containing an inorganic compound, and then the oligomer and/or the prepolymer is washed, it is possible to prepare an organic acid oligomer, prepolymer, and polymer having high purity and biodegradability, similarly to a conventional process of polymerizing an organic acid of high purity, and it was confirmed that a polymer having a high molecular weight may be prepared, and completed the invention.

The organic acid composition containing an inorganic compound may be recovered from a fermentation broth obtained by fermenting a strain capable of producing an organic acid. However, the organic acid composition containing an inorganic compound may contain a large amount of inorganic impurities such as an inorganic compound because it does not go through an additional mineral purification process, and for example, the purity of the organic acid in the organic acid composition containing an inorganic compound may be 90% or less, 88% or less, 85% or less, 83% or less, 80% or less, 75% or less, 70% or less, 65% or less, 50% or less, or 5% or more and 40% or less.

The organic acid is not particularly limited as long as it may be produced from microbial fermentation, but may be, for example, one or more selected from the group consisting of lactic acid, 3-hydroxypropionic acid, butyric acid, acetic acid, propionic acid, succinic acid, and salts thereof, and may be lactic acid or 3-hydroxypropionic acid in order to prepare a high value-added oligomer, prepolymer, polymer, and the like.

The method for preparing an organic acid prepolymer according to the one embodiment may further comprise a step of producing an organic acid fermentation broth by fermenting a strain capable of producing an organic acid, before the step of preparing the oligomer and/or the prepolymer by polymerizing the organic acid composition containing an inorganic compound.

A microorganism such as a strain capable of producing an organic acid can produce an organic acid fermentation broth by fermenting a low molecular weight sugar. Such a microorganism may be a natural microorganism or an engineered microorganism, and the microorganism may be, for example, a bacterium, such as a cellulose-degrading bacterium, a fungus, such as a yeast, a plant, or a protist, such as an alga, a protozoan, or a fungus-like protist, such as a slime mold, and if an organism does not cause a rejection reaction, a mixture of organisms may be used.

For example, a strain having the ability to produce 3-hydroxypropionic acid may be one containing a gene encoding one or more proteins selected from the group consisting of a glycerol dehydratase and an aldehyde dehydrogenase, or may be one containing a gene encoding the two proteins. In one example, the 3-hydroxypropionic acid-producing strain may further comprise a gene (gdrAB) encoding a glycerol dehydratase reactivating enzyme (GdrAB). In one example, the 3-hydroxypropionic acid-producing strain may further be a strain capable of biosynthesizing vitamin B₁₂.

The glycerol dehydratase may be one encoded by the dhaB (GenBank accession no. U30903.1) gene, but is not limited thereto. The dhaB gene may be an enzyme derived from Klebsiella pneumonia, but is not limited thereto. The gene encoding the glycerol dehydratase may comprise a gene encoding dhaB1, dhaB2, and/or dhaB3. The glycerol dehydratase protein and the gene encoding it may include variations in the gene and/or amino acid sequence within a range that maintains the enzymatic activity of decomposing glycerol into 3-hydroxypropanal (3-HPA) and water (H₂O).

The gene (aldH) encoding the aldehyde dehydrogenase (ALDH) may be, for example, an aldH (GenBank Accession no. U00096.3; EaldH) gene derived from Escherichia coli or an E. coli K12 MG1655 cell line, a puuC gene derived from K. pneumonia, and/or a KGSADH gene derived from Azospirillum brasilense, but is not limited thereto. The aldehyde dehydrogenase protein and the gene encoding it may include variations in the gene and/or amino acid sequence within a range that maintains the activity for producing 3-hydroxypropionic acid from 3-hydroxypropanal.

The medium for producing the fermentation broth may be selected without limitation within the scope of the purpose for producing the organic acid. In one example, the medium may be one containing glycerol as a carbon source. In another example, the medium may be crude glycerol and/or pretreated crude glycerol, but is not limited thereto. In one example, the production medium may further comprise vitamin B₁₂.

In the step of producing the organic acid fermentation broth by fermenting the strain capable of producing an organic acid, the concentration of the organic acid contained in the organic acid fermentation broth may be 1 g/L or more and 200 g/L or less, 10 g/L or more and 150 g/L or less, 30 g/L or more and 130 g/L or less, or 40 g/L or more and 100 g/L or less.

In addition, the fermentation may be a neutral fermentation, and for example, the pH during fermentation may be maintained in the range of 6.0 or more and 8.0 or less, 6.5 or more and 7.5 or less, or 6.5 or more and 7.5 or less, but is not limited thereto. The pH range may be appropriately adjusted as necessary. The alkali metal salt may be added for the neutral fermentation. The alkali metal salt may be one containing Mg²⁺, Ca²⁺, or a mixture thereof. In addition, the alkali metal salt may be Ca(OH)₂ or Mg(OH)₂, but is not limited thereto.

The method for preparing an organic acid prepolymer according to the one embodiment may further comprise a step of precipitating an organic acid salt crystal by mixing the fermentation broth and an organic solvent after the step of producing the organic acid fermentation broth.

In order to recover the organic acid salt crystal from the fermentation broth, after dropping the organic solvent into the fermentation broth or dropping the fermentation broth into the organic solvent, the fermentation broth and the organic solvent are mixed, and particle formation of the organic acid salt crystal is induced to precipitate the organic acid salt. The precipitation may be carried out at a temperature of -10 °C to 40 °C, -9 °C to 35 °C, -8 °C to 30 °C, -7 °C to 30 °C, -6 °C to 25 °C, -5 °C to 20 °C, or 0 °C to 15 °C.

The organic solvent is not limited thereto, but may be, for example, an alcohol, water, an ether, a ketone, a halogenated hydrocarbon, an aromatic hydrocarbon, or the like.

The alcohol may be, for example, one or more selected from the group consisting of a straight-chain or branched-chain alcohol having 1 to 4 carbon atoms, such as methanol, ethanol, and propanol (e.g., isopropyl alcohol).

The ether may be ethyl ether, dioxane, tetrahydrobutane, or the like, the ketone may be acetone, methyl ethyl ketone, methyl butyl ketone, methyl isobutyl ketone, or the like, the halogenated hydrocarbon may be dichloromethane, chloroform, dichloroethane, trichloroethane, tetrachloroethane, dichloroethylene, trichloroethylene, tetrachloroethylene, or the like, and the aromatic hydrocarbon may be benzene, toluene, xylene, or the like.

In addition, the organic solvent may have a concentration of 10 to 100% (v/v), 20 to 100 (v/v), 30 to 100 (v/v), 40 to 100 (v/v), 50 to 100 (v/v), 60 to 100 (v/v), 70 to 100 (v/v), 80 to 100 (v/v), 90 to 100 (v/v), 95 to 100 (v/v), or 98 to 100 (v/v), for example, 99% (v/v).

The amount of the organic solvent added may be 5 to 10 times, 6 to 9 times, or 7 to 8 times that of the fermentation broth.

The method for preparing an organic acid prepolymer according to the one embodiment may further comprise a step of preparing a solution in which the organic acid salt crystal is dissolved and adding an acid to control the pH to 5 or less, after the step of precipitating the organic acid salt crystal by mixing the fermentation broth and the organic solvent.

For example, after precipitating the organic acid salt crystal, the organic acid salt crystal may be filtered, a solution in which the filtered organic acid salt crystal is dissolved may be prepared, and an acid may be added to control the pH to 5 or less.

In addition, in the step of adding the acid, the organic acid may be protonated by preparing a salt by titrating the acid. At this time, the acid is not particularly limited as long as it is an acid that can control the pH of the fermentation broth to 5 or less, but may be, for example, one selected from the group consisting of sulfuric acid, hydrochloric acid, phosphoric acid, carbonic acid, and nitric acid. In addition, the salt prepared by acid titration may be CaSO₄(s) or MgSO₄(s), but is not limited thereto. In addition, by adding an acid to the fermentation broth, the pH of the fermentation broth may be controlled to 5.0 or less, 4.0 or less, 3.0 or less, 2.0 or less, 1.0 or less, or 0.5 or less.

The method for preparing an organic acid prepolymer comprises a step of preparing an oligomer and/or a prepolymer by polymerizing an organic acid composition containing an inorganic compound, and the organic acid composition containing an inorganic compound may contain inorganic impurities such as an inorganic compound depending on the metal salt used during fermentation, and is not limited thereto, but for example, the organic acid composition containing an inorganic compound may contain magnesium, calcium, sodium, phosphorus, sulfur, potassium, chlorine, salts thereof, derivatives thereof, or a mixture thereof.

In addition, the organic acid composition containing an inorganic compound may contain one or more elements selected from the group consisting of a magnesium element, a calcium element, a sodium element, a phosphorus element, a sulfur element, a potassium element, and a chlorine element, and the content of the element may be 5.0 wt% or less, for example, 3.0 wt% or less, 2.0 wt% or less, or 1.0 wt% or less, based on 100 wt% of the organic acid composition containing an inorganic compound.

For example, the organic acid composition containing an inorganic compound may have a concentration of a calcium element (Ca) of 50 ppm or more, 100 ppm or more, 200 ppm or more, or 300 ppm or more and 3,000 ppm or less.

In addition, the organic acid composition containing an inorganic compound may have a concentration of a sodium element (Na) of 300 ppm or more, 500 ppm or more, 700 ppm or more, 1,000 ppm or more, 1,300 ppm or more, 1,500 ppm or more, or 1,800 ppm or more and 3,000 ppm or less.

In addition, the organic acid composition containing an inorganic compound may have a concentration of a phosphorus element (P) of 1 ppm or more, 5 ppm or more, 8 ppm or more, or 10 ppm or more and 3,000 ppm or less.

In addition, the organic acid composition containing an inorganic compound may have a concentration of a sulfur element (S) of 300 ppm or more, 500 ppm or more, 700 ppm or more, 1,000 ppm or more, 1,300 ppm or more, 1,500 ppm or more, or 1,800 ppm or more and 3,000 ppm or less.

In addition, the organic acid composition containing an inorganic compound may have a concentration of a potassium element (K) of 50 ppm or more, 100 ppm or more, 200 ppm or more, or 300 ppm or more and 3,000 ppm or less.

In addition, the organic acid composition containing an inorganic compound may have a concentration of a chlorine element (CI) of 300 ppm or more, 500 ppm or more, 700 ppm or more, 1,000 ppm or more, 1,500 ppm or more, 2,000 ppm or more, 2,500 ppm or more, or 3,500 ppm or more and 10,000 ppm or less.

Meanwhile, the element concentration may be measured using ICP-OES (Inductively Coupled Plasma - Optical Emission Spectrometry).

In the step of preparing the oligomer and/or the prepolymer by polymerizing the organic acid composition containing an inorganic compound, the polymerization may be carried out at a temperature of 75 °C or more and 200 °C or less, for example, 80 °C or more, 85 °C or more, 90 °C or more, 120 °C or more, 150 °C or more, or 200 °C or more, and 180 °C or less, 160 °C or less, 150 °C or less, 130 °C or less, 125 °C or less, 120 °C or less, 115 °C or less, or 110 °C or less. If the polymerization temperature is too low, the molecular weight of the oligomer and/or the prepolymer may be low, and if the polymerization temperature is too high, it may be difficult for the polymerization of the organic acid to proceed.

In addition, the polymerization of the organic acid composition containing an inorganic compound may be carried out at a pressure of 1 torr or more and 760 torr or less, for example, 1 torr or more, 5 torr or more, 7 torr or more, 10 torr or more, 50 torr or more, 100 torr or more, 200 torr or more, or 300 torr or more, and 700 torr or less, 500 torr or less, 300 torr or less, 200 torr or less, 150 torr or less, 100 torr or less, 70 torr or less, or 50 torr or less, for 1 hour or more and 10 hours or less. In addition, the reaction time of the polymerization of the organic acid composition containing an inorganic compound may be appropriately considered in consideration of the molecular weight, yield, etc. of the produced oligomer and prepolymer, and is preferably performed for 1 hour to 10 hours, 2 hours to 8 hours, or 3 hours to 5 hours.

In addition, the polymerization of the organic acid composition containing an inorganic compound may be carried out without a catalyst or in the presence of a tin-based catalyst and/or a sulfonic acid-based catalyst. The tin-based catalyst may be dibutyl tin dilaurate (DBTDL), dioctyl tin dilaurate (DOTDL), dibutyl tin diacetate, stannous acetate, or tin caprylate, and the sulfonic acid-based catalyst may be, for example, p-toluenesulfonic acid, m-xylene-4-sulfonic acid, 2-mesitylenesulfonic acid, or p-xylene-2-sulfonic acid. The catalyst may be used in an amount of 0.05 mol% or more and 0.5 mol% or less, 0.1 mol% or more and 0.4 mol% or less, or 0.2 mol% or more and 0.4 mol% or less based on the organic acid.

The oligomer and the prepolymer prepared by the polymerization have a weight-average molecular weight of 800 or more, 1,000 or more, 1,300 or more, 1,500 or more, 2,000 or more, 2,500 or more, or 3,000 or more, and 15,000 or less, 10,000 or less, 9,000 or less, 8,000 or less, or 7,000 or less.

In addition, the oligomer and the prepolymer have a number-average molecular weight of 700 or more, 800 or more, 900 or more, 1,000 or more, 1,200 or more, 1,500 or more, or 2,000 or more, and 15,000 or less, 10,000 or less, 9,000 or less, 8,000 or less, or 7,000 or less.

The method for preparing an organic acid prepolymer according to the one embodiment may comprise a step of washing the oligomer and/or the prepolymer with a washing solution.

The washing solution may be water, distilled water, or a solution containing a basic compound, for example, aqueous ammonia. In addition, the basic compound is not particularly limited as long as it is a basic compound not containing a metal ion, but examples thereof include a quaternary ammonium compound, an amine, and the like.

Examples of the quaternary ammonium compound include tetramethylammonium hydroxide (TMAH), trimethyl-2-hydroxyethylammonium hydroxide (choline), tetraethylammonium hydroxide, tetrapropylammonium hydroxide, tetrabutylammonium hydroxide, trimethylphenylammonium hydroxide, benzyltrimethylammonium hydroxide, and the like.

The amine may be a primary aliphatic amine, a secondary aliphatic amine, a tertiary aliphatic amine, or a cycloaliphatic amine, and for example, the primary aliphatic amine may be monoethanolamine, ethylenediamine, 2-(2-aminoethoxyethanol), 2-(2-aminoethylamino)ethanol, diethylenetriamine, triethylenetetramine, or the like, the secondary aliphatic amine may be diethanolamine, N-methylaminoethanol, N-hydroxyethylaminoethanol, dipropylamine, 2-ethylaminoethanol, or the like, the tertiary aliphatic amine may be triethanolamine, dimethylaminoethanol, ethyldiethanolamine, or the like, and the cycloaliphatic amine may be cyclopentylamine, cyclohexylamine, or the like.

The content of the basic compound contained in the washing solution may be contained in an amount of 1 wt% or more, 3 wt% or more, 5 wt% or more, 7 wt% or more, 8 wt% or more, or 10 wt% or more, and 30 wt% or less, 25 wt% or less, 20 wt% or less, 15 wt% or less, or 10 wt% or less, based on a total of 100 wt% of the washing solution.

When washing the oligomer and/or the prepolymer with the washing solution, the washing temperature may be 50 °C or more and 95 °C or less, for example, washing may be performed at a temperature of 55 °C or more, 60 °C or more, or 70 °C or more, and washing may be performed at a temperature of 93 °C or less, 90 °C or less, 85 °C or less, 80 °C or less, or 75 °C or less.

If the washing temperature is too low, the prepolymer may not be dissolved and washing may be difficult, and if the washing temperature is too high, the oligomer and/or the prepolymer may be thermally decomposed.

When washing the oligomer and/or the prepolymer with the washing solution, the washing solution may be used in a weight of 0.5 times or more and 30 times or less based on the weight of the oligomer and/or the prepolymer, for example, in a weight of 0.7 times or more, 1.0 time or more, 2.0 times or more, 3.0 times or more, or 4.0 times or more, and in a weight of 28 times or less, 25 times or less, 23 times or less, or 20 times or less.

In addition, the purity of the oligomer and/or the prepolymer washed by the washing process may be 90% or more, 93% or more, 95% or more, 98% or more, 99% or more, or 100%.

The method for preparing an organic acid prepolymer according to the one embodiment may further comprise a step of solid-liquid or liquid-liquid separating the washed oligomer and/or the prepolymer from the washing solution after the step of washing the oligomer and/or the prepolymer with the washing solution.

The solid-liquid separating process may be separated through filtration or the like, and the washed oligomer and/or the prepolymer and the washing solution may be separated using a filtration flask and a vacuum pump.

In addition, since the oligomer and/or the prepolymer in a molten state has hydrophobicity or reduced hydrophilicity unlike the hydrophilic washing solution and may be separated from the washing solution, in the liquid-liquid separating process, the oligomer and/or the prepolymer may be recovered by centrifuging to separate an upper layer liquid and a lower layer liquid.

In addition, the washing solution recovered through the separation process may be reused in an additional washing process.

According to another embodiment of the invention, there is provided an organic acid prepolymer composition comprising an organic acid prepolymer in which an organic acid composition containing an inorganic compound is polymerized, wherein the inorganic compound is removed by washing.

At this time, the organic acid prepolymer may be a 3-hydroxypropionic acid prepolymer. In addition, the organic acid prepolymer composition may be one prepared by the method for preparing an organic acid prepolymer according to the one embodiment.

Meanwhile, the prepolymer may be a meaning including an oligomer.

In addition, the organic acid composition containing an inorganic compound is as described above in the method for preparing an organic acid prepolymer according to the one embodiment, and may be, for example, one recovered from a fermentation broth obtained by fermenting a strain capable of producing an organic acid. Such a fermentation broth may contain a large amount of inorganic impurities such as an inorganic compound, and the organic acid prepolymer composition may have a high purity because such an inorganic compound is removed by washing. In addition, in addition to the by-products of the fermentation broth, the catalyst used during polymerization may be removed by the washing, thereby exhibiting a high purity. For example, the purity of the organic acid prepolymer may be 90% or more, 93% or more, 95% or more, 98% or more, 99% or more, or 100%.

For example, the washed organic acid prepolymer may have a concentration of a calcium element (Ca) of 200 ppm or less, 150 ppm or less, 100 ppm or less, 50 ppm or less, 30 ppm or less, 15 ppm or less, or 1 ppm or more and 10 ppm or less.

In addition, the washed organic acid prepolymer may have a concentration of a sodium element (Na) of 1,000 ppm or less, 500 ppm or less, 100 ppm or less, 50 ppm or less, 20 ppm or less, or 1 ppm or more and 15 ppm or less.

In addition, the washed organic acid prepolymer may have a concentration of a phosphorus element (P) of 50 ppm or less, 30 ppm or less, 15 ppm or less, or 1 ppm or more and 10 ppm or less.

In addition, the washed organic acid prepolymer may have a concentration of a sulfur element (S) of 1,000 ppm or less, 500 ppm or less, 300 ppm or less, 200 ppm or less, 100 ppm or less, or 1 ppm or more and 80 ppm or less.

In addition, the washed organic acid prepolymer may have a concentration of a potassium element (K) of 200 ppm or less, 100 ppm or less, 50 ppm or less, 30 ppm or less, 15 ppm or less, or 1 ppm or more and 10 ppm or less.

In addition, the washed organic acid prepolymer may have a concentration of a chlorine element (CI) of 1,000 ppm or less, 500 ppm or less, 100 ppm or less, 50 ppm or less, 20 ppm or less, 15 ppm or less, or 1 ppm or more and 10 ppm or less.

Meanwhile, the element concentration may be measured using ICP-OES (Inductively Coupled Plasma - Optical Emission Spectrometry).

In addition, the washing is as described above in the method for preparing an organic acid prepolymer according to the one embodiment.

The prepolymer may have a weight-average molecular weight of 800 or more, 1,000 or more, 1,300 or more, 1,500 or more, 2,000 or more, 2,500 or more, or 3,000 or more, and 15,000 or less, 10,000 or less, 9,000 or less, 8,000 or less, or 7,000 or less, and a number-average molecular weight of 700 or more, 800 or more, 900 or more, 1,000 or more, 1,200 or more, 1,500 or more, or 2,000 or more, and 15,000 or less, 10,000 or less, 9,000 or less, 8,000 or less, or 7,000 or less.

According to still another embodiment of the invention, there is provided a method for preparing an organic acid polymer, comprising: a step of preparing an oligomer and/or a prepolymer by polymerizing an organic acid composition containing an inorganic compound; a step of washing the oligomer and/or the prepolymer with a washing solution; and a step of preparing a polymer by polymerizing the washed oligomer and/or the prepolymer.

The step of preparing the oligomer and/or the prepolymer by polymerizing the organic acid composition containing an inorganic compound and the step of washing the oligomer and/or the prepolymer with the washing solution are as described above in the method for preparing an organic acid prepolymer according to the one embodiment.

In the step of preparing the polymer by polymerizing the washed oligomer and/or the prepolymer, the polymerization may be carried out at a temperature of 90 °C or more and 200 °C or less and a pressure of 0.01 torr or more and 760 torr or less for 20 hours or more and 100 hours or less. For example, the reaction temperature of the polymerization may be 95 °C or more and 180 °C or less, 100 °C or more and 150 °C or less, or 105 °C or more and 115 °C or less. In addition, the pressure of the polymerization is 700 torr or less, 500 torr or less, 300 torr or less, 200 torr or less, 100 torr or less, 50 torr or less, 40 torr or less, or 30 torr or less, and 0.01 torr or more, 0.02 torr or more, 0.03 torr or more, 0.04 torr or more, 0.05 torr or more, 0.06 torr or more, 0.07 torr or more, 0.08 torr or more, 0.09 torr or more, or 0.10 torr or more. The reaction time of the polymerization may be appropriately considered in consideration of the molecular weight, yield, etc. of the produced polymer, and is preferably performed for 20 hours to 90 hours, 40 hours to 80 hours, or 50 hours to 70 hours.

In addition, the polymerization may be carried out in the presence of a tin-based catalyst and/or a sulfonic acid-based catalyst. The tin-based catalyst may be dibutyl tin dilaurate (DBTDL), dioctyl tin dilaurate (DOTDL), dibutyl tin diacetate, stannous acetate, or tin caprylate, and the sulfonic acid-based catalyst is, for example, p-toluenesulfonic acid, m-xylene-4-sulfonic acid, 2-mesitylenesulfonic acid, or p-xylene-2-sulfonic acid. The catalyst may be used in an amount of 0.05 mol% or more and 0.5 mol% or less, 0.1 mol% or more and 0.4 mol% or less, or 0.2 mol% or more and 0.4 mol% or less based on the organic acid and/or the prepolymer.

According to still another embodiment of the invention, there is provided an organic acid polymer in which the organic acid prepolymer composition is polymerized.

The organic acid polymer may be a 3-hydroxypropionic acid polymer.

In addition, the organic acid prepolymer composition may be one prepared by the method for preparing an organic acid prepolymer according to the one embodiment.

The organic acid polymer may have a weight-average molecular weight of 10,000 or more, and may be 11,000 or more, 15,000 or more, 17,000 or more, 20,000 or more, or 21,000 or more, and 40,000 or less, 39,000 or less, 38,000 or less, 37,000 or less, 36,000 or less, or 35,000 or less.

In addition, the organic acid polymer may have a number-average molecular weight of 7,000 or more, and for example, may be 7,100 or more, 7,200 or more, 7,300 or more, 7,400 or more, 8,000 or more, 8,500 or more, 9,000 or more, or 9,500 or more, and 23,000 or less, 22,000 or less, 21,000 or less, 20,000 or less, or 19,000 or less.

### ADVANTAGEOUS EFFECTS

According to the present invention, it is possible to provide a method for preparing an oligomer, a prepolymer, and a polymer of high purity and high molecular weight from an organic acid composition of low purity containing an inorganic compound, efficiently and at a low process cost.

### DETAILED DESCRIPTION OF THE EMBODIMENTS

Hereinafter, embodiments of the present invention will be described in more detail in the following examples. However, the following examples are merely for illustrating the embodiments of the present invention, and the content of the present invention is not limited by the following examples.

### Preparation Example 1. Production of 3-hydroxypropionic acid

A recombinant vector was prepared by introducing a gene encoding a glycerol dehydratase, which is known to produce 3-hydroxypropionic acid (3-HP) using glycerol as a substrate, and an aldehyde dehydrogenase. The prepared recombinant vector was introduced into an Escherichia coli (E.coli) W3110 strain to construct a 3-hydroxypropionic acid-producing strain.

More specifically, a pCDF_J23101_dhaB_gdrAB_J23100_aldH_btuR vector obtained by cloning a BtuR gene encoding adenosyltransferase into a plasmid pCDF containing a gene (dhaB) encoding glycerol dehydratase, a gene (aldH) encoding aldehyde dehydrogenase, and a gene (gdrAB) encoding glycerol dehydratase reactivase was introduced into a W3110 strain (KCCM 40219) by electroporation using an electroporator (Bio-Rad, Gene Pulser Xcell) to construct a 3-hydroxypropionic acid-producing strain. The process for constructing the 3-hydroxypropionic acid-producing strain of this Preparation Example 1 and the vectors, primers, and enzymes used were performed with reference to Example 1 of Korean Laid-open Patent No. 10-2020-0051375.

The prepared strain for producing 3-hydroxypropionic acid was fermented and cultured at 35°C in a 5L fermenter using unpurified glycerol as a carbon source to produce 3-hydroxypropionic acid. To prevent a decrease in pH due to the production of 3-hydroxypropionic acid, calcium hydroxide (Ca(OH)₂), an alkali metal salt, was added to maintain the pH during fermentation at neutral.

After fermentation and culture, cells were removed by centrifugation, and organic impurities were purified to prepare a 3-hydroxypropionic acid solution (concentration 5-30 g/L).

### Example 1

### (1) Preparation of prepolymer

500 g of the 3-hydroxypropionic acid solution (content 11.7%) prepared in Preparation Example 1 was added to a 1000 ml glass reactor, and vacuum distillation was performed at a temperature of 40 to 50 °C to prepare a concentrated 3-hydroxypropionic acid aqueous solution (content 72%). Solid precipitation during concentration was further removed by filtration. 80 g of the 3-hydroxypropionic acid aqueous solution and 0.5 g of p-toluenesulfonic acid (p-TSA) catalyst were added to an oil bath, and a polymerization reaction was carried out at a temperature of 90 °C and a pressure of 70 torr for 2 hours to prepare a 3-hydroxypropionic acid prepolymer.

### (2) Washing of prepolymer

The 3-hydroxypropionic acid prepolymer was washed with a washing solution at a temperature of 85 °C using water. At this time, the washing solution was used in a weight of 3 times that of the 3-hydroxypropionic acid prepolymer for washing. Thereafter, using a filtration flask and a vacuum pump, the washed prepolymer and the washing solution were subjected to solid-liquid separation to recover the washed prepolymer, and the prepolymer was dried in an oven at a temperature of 40 °C for 20 hours to finally recover the dried prepolymer.

### (3) Preparation of polymer

30 g of the prepolymer and 0.25 g of p-toluenesulfonic acid (p-TSA) catalyst were added to an oil bath, and polymerized at a temperature of 95 °C and a pressure of 1 torr for 72 hours to prepare a polymer, that is, poly(3-hydroxypropionate).

### Example 2

### (1) Preparation of prepolymer

500 g of the 3-hydroxypropionic acid solution (content 11.7%) prepared in Preparation Example 1 was added to a 1000 ml glass reactor, and vacuum distillation was performed at a temperature of 40 to 50 °C to prepare a concentrated 3-hydroxypropionic acid aqueous solution (content 72%). 80 g of the 3-hydroxypropionic acid aqueous solution and 0.5 g of p-toluenesulfonic acid (p-TSA) catalyst were added to an oil bath, and a polymerization reaction was carried out at a temperature of 90 °C and a pressure of 70 torr for 2 hours to prepare a 3-hydroxypropionic acid prepolymer.

### (2) Washing of prepolymer

A washing solution was prepared by mixing 500 ml of water and 5 ml of aqueous ammonia, and the 3-hydroxypropionic acid prepolymer was washed with the washing solution at a temperature of 85 °C. At this time, the washing solution was used in a weight of 1 time that of the 3-hydroxypropionic acid prepolymer. Thereafter, using a filtration flask and a vacuum pump, the washed prepolymer and the washing solution were subjected to liquid-liquid separation to recover the washed prepolymer.

### (3) Preparation of polymer

30 g of the prepolymer and 0.25 g of p-toluenesulfonic acid (p-TSA) catalyst were added to an oil bath, and polymerized at a temperature of 90 °C and a pressure of 1 torr for 30 hours to prepare a polymer, that is, poly(3-hydroxypropionate).

### Example 3

### (1) Preparation of prepolymer

500 g of the 3-hydroxypropionic acid solution (content 11.7%) prepared in Preparation Example 1 was added to a 1000 ml glass reactor, and vacuum distillation was performed at a temperature of 40 to 50 °C to prepare a concentrated 3-hydroxypropionic acid aqueous solution (content 72%). 80 g of the 3-hydroxypropionic acid aqueous solution and 0.5 g of p-toluenesulfonic acid (p-TSA) catalyst were added to an oil bath, and a polymerization reaction was carried out at a temperature of 90 °C and a pressure of 70 torr for 2 hours to prepare a 3-hydroxypropionic acid prepolymer.

### (2) Washing of prepolymer

The 3-hydroxypropionic acid prepolymer was washed twice with double distilled water at a temperature of 85 °C. At this time, the washing solution was used in a weight of 2 times that of the 3-hydroxypropionic acid prepolymer. Thereafter, using a filtration flask and a vacuum pump, the washed prepolymer and the washing solution were subjected to liquid-liquid separation to recover the washed prepolymer.

### (3) Preparation of polymer

30 g of the prepolymer and 0.25 g of p-toluenesulfonic acid (p-TSA) catalyst were added to an oil bath, and polymerized at a temperature of 90 °C and a pressure of 1 torr for 30 hours to prepare a polymer, that is, poly(3-hydroxypropionate).

### Comparative Example 1

A prepolymer and a polymer were prepared in the same manner as in Example 1, except that the (2) prepolymer washing process was not performed.

### Reference Example 1

### (1) Purification of 3-hydroxypropionic acid

The inorganic impurities remaining in the 3-hydroxypropionic acid solution prepared in Preparation Example 1 were purified by electrodialysis using an electrodialysis apparatus from Inomeditech. Specifically, the electrodialysis cell of the electrodialysis apparatus used an L3 membrane from Inomeditech as the cation and anion separation membrane, and was operated in a constant voltage mode of 20 cells (20V, 1V per cell) for 40 minutes. At this time, electrodialysis was performed until the electrical conductivity of the desalination chamber decreased to 10% compared to the initial conductivity of 100% of the desalination chamber to recover 3-hydroxypropionic acid.

### (2) Preparation of prepolymer

600 g of the 3-hydroxypropionic acid solution (content 10%) prepared in Preparation Example 1 was added to a 1000 ml glass reactor, and vacuum distillation was performed at a temperature of 40 to 50 °C to prepare concentrated 3-hydroxypropionic acid (content 72%). 80 g of the 3-hydroxypropionic acid aqueous solution (72%) and 0.5 g of p-toluenesulfonic acid (p-TSA) catalyst were added to an oil bath, and a polymerization reaction was carried out at a temperature of 90 °C and a pressure of 70 torr for 2 hours to prepare a 3-hydroxypropionic acid prepolymer.

### (3) Preparation of polymer

Without separate transfer, the temperature of the oil bath was raised to 90 °C and the prepolymer was polymerized at a pressure of 1 torr for 21 hours to prepare a polymer, that is, poly(3-hydroxypropionate).

### Evaluation

### 1. GPC (gel permeation chromatography) molecular weight evaluation

For each of the prepolymers and polymers prepared in each step in the above Examples and Comparative Examples, the molecular weight was evaluated using a Water e2695 model instrument and Agilent Plgel mixed c and b columns. A sample was prepared at 4 mg/ml with chloroform as a solvent, and 20 ul was injected. The weight-average molecular weight (Mw), number-average molecular weight (Mn), and polydispersity index (PDI) were measured by the measured gel permeation chromatography (GPC: Tosoh ECO SEC Elite), and the results are shown in Table 1 below. Meanwhile, cases where the molecular weight of the prepolymer was not measured are indicated by '-' in Table 1.
Solvent: chloroform (eluent)
Flow rate: 1.0 ml/min
Column temperature: 40 °C
Standard: Polystyrene (calibrated with a cubic function)

### 2. Element content measurement

The concentration of elements contained in the prepolymers prepared in Example 1 and Comparative Example 1 was measured using ICP-OES (Inductively Coupled Plasma - Optical Emission Spectrometry), and the results are shown in Table 1 below.

Specifically, pretreatment was carried out by Prep. Acid Digestion, and about 0.05 g of a sample was accurately weighed in a Corning tube, 0.5 mL of nitric acid was added to the sample, and the sample was dissolved by shaking overnight at room temperature. To accelerate the reaction of the sample, a small amount of hydrogen peroxide was added to dissolve the sample, and when the sample was completely dissolved and clea r, it was diluted to 10 mL with tertiary ultrapure water to prepare a sample for analysis. After removing undissolved components with a 0.45 µm PTFE filter, the remaining filtrate was injected into an ICP-OES instrument (AVIO 500, Perkin Elmer) to perform component analysis. Meanwhile, cases where the element concentration was not measured are indicated by '-' in Table 1.

### <ICP-OES analysis conditions>

RF Power: 1300 W
Torch Height: 15 mm
Plasma gas flow rate: 15 L/min
Sample gas flow rate: 0.8 L/min
Auxiliary gas flow rate: 0.20 L/min
Pump speed: 1.5 ml/min

**[Table 1]**

| | Element Concentration (ppm) | | | | | | Molecular Weight of prepolymer (Da) | | | Molecular Weight of polymer (Da) | | |
|---|---|---|---|---|---|---|---|---|---|---|---|---|
| | Ca | Na | P | S | K | Cl | Mn | Mw | PDI | Mn | Mw | PDI |
| Example 1 | <10 | 14 | <10 | 76 | <10 | 7 | 2440 | 2156 | 1.3 | 7,420 | 11,040 | 1.49 |
| Example 2 | - | - | - | - | - | - | - | - | - | 11,020 | 21,010 | 1.90 |
| Example 3 | - | - | - | - | - | - | - | - | - | 9,967 | 23,220 | 2.32 |
| Comparative Example 1 | 320 | 1850 | <10 | 1421 | 356 | 3200 | 2120 | 3230 | 1.5 | 2,120 | 3,230 | 1.52 |
| Reference Example 1 | - | - | - | - | - | - | - | - | - | 10,556 | 24,010 | 2.27 |

Referring to Table 1, it was confirmed that in Example 1, where the washing process was performed after polymerization into a prepolymer, the concentration of elements such as inorganic elements was low, confirming that a large amount of inorganic impurities such as inorganic compounds was removed from the starting material "organic acid composition containing inorganic compound" through the washing process, whereas in the prepolymer of Comparative Example 1, where washing was not performed, a large amount of elements such as inorganic elements was included, confirming that inorganic impurities such as inorganic compounds from the starting material "organic acid composition containing inorganic compound" were still present in the prepolymer. In addition, it was confirmed that Examples 1 to 3, in which a polymer was prepared by polymerizing the washed prepolymer, had a higher number-average molecular weight and weight-average molecular weight than Comparative Example 1, in which a polymer was prepared by polymerizing the unwashed prepolymer. In addition, it was confirmed that Examples 1 to 3 had a polymer molecular weight similar to that of Reference Example 1, in which the organic acid was purified through an electrodialysis apparatus.

## Claims

1. A method for preparing an organic acid prepolymer, comprising:
a step of preparing an oligomer and/or a prepolymer by polymerizing an organic acid composition containing an inorganic compound; and
a step of washing the oligomer and/or the prepolymer with a washing solution.

2. The method for preparing an organic acid prepolymer of Claim 1,
wherein the organic acid is one or more selected from the group consisting of lactic acid, 3-hydroxypropionic acid, butyric acid, acetic acid, propionic acid, succinic acid, and salts thereof.

3. The method for preparing an organic acid prepolymer of Claim 1,
wherein the purity of the organic acid in the organic acid composition containing an inorganic compound is 90% or less.

4. The method for preparing an organic acid prepolymer of Claim 1,
wherein the organic acid composition containing an inorganic compound comprises magnesium, calcium, sodium, phosphorus, sulfur, potassium, chlorine, salts thereof, derivatives thereof, or a mixture thereof.

5. The method for preparing an organic acid prepolymer of Claim 1,
wherein the oligomer and the prepolymer each independently have a weight-average molecular weight of 800 or more and 15,000 or less.

6. The method for preparing an organic acid prepolymer of Claim 1,
wherein the organic acid composition containing an inorganic compound is recovered from a fermentation broth obtained by fermenting a strain capable of producing an organic acid.

7. The method for preparing an organic acid prepolymer of Claim 1, further comprising,
before the step of preparing the oligomer and/or the prepolymer by polymerizing the organic acid composition containing an inorganic compound,
a step of producing an organic acid fermentation broth by fermenting a strain capable of producing an organic acid.

8. The method for preparing an organic acid prepolymer of Claim 7, further comprising:
a step of precipitating an organic acid salt crystal by mixing the fermentation broth and an organic solvent.

9. The method for preparing an organic acid prepolymer of Claim 8, further comprising:
a step of preparing a solution in which the organic acid salt crystal is dissolved, and adding an acid to control the pH to 5 or less.

10. The method for preparing an organic acid prepolymer of Claim 1,
wherein the washing solution is water or a solution comprising a basic compound.

11. The method for preparing an organic acid prepolymer of Claim 1,
wherein the washing is carried out at a temperature of 50 °C or more and 300 °C or less.

12. The method for preparing an organic acid prepolymer of Claim 1, further comprising,
after the step of washing the oligomer and/or the prepolymer with a washing solution,
a step of solid-liquid or liquid-liquid separating the washed oligomer and/or the prepolymer and the washing solution.

13. A method for preparing an organic acid polymer, comprising:
a step of preparing an oligomer and/or a prepolymer by polymerizing an organic acid composition containing an inorganic compound;
a step of washing the oligomer and/or the prepolymer with a washing solution; and
a step of preparing a polymer by polymerizing the washed oligomer and/or the prepolymer.

14. The method for preparing an organic acid polymer of Claim 13,
wherein the polymer has a number-average molecular weight of 7,000 or more.

15. The method for preparing an organic acid polymer of Claim 13,
wherein the polymer has a weight-average molecular weight of 10,000 or more.

16. An organic acid prepolymer composition, comprising an organic acid prepolymer in which an organic acid composition containing an inorganic compound is polymerized,
wherein the inorganic compound is removed by washing.

17. The organic acid prepolymer composition of Claim 16,
wherein the organic acid prepolymer is a 3-hydroxypropionic acid prepolymer.

18. An organic acid polymer, wherein the organic acid prepolymer composition according to Claim 16 is polymerized.

19. The organic acid polymer of Claim 18,
wherein the organic acid polymer is a 3-hydroxypropionic acid polymer.
